# EUROPEAN PATENT APPLICATION

(11) **EP 2 927 830 A1**
(43) Date of publication of application: **07.10.2015**
(21) Application number: 15161663.8
(22) Date of filing: 30.03.2015
(51) Int. Cl.: G06F 19/00, A61B 6/00, A61B 1/00

(54) **Inspection report creation support system, medical image diagnosis apparatus, inspection report creation support method, and program**

(30) Priority: 31.03.2014 JP 2014072717
(71) Applicant: Fujifilm Corporation, Minato-ku Tokyo (JP)
(72) Inventor: Miura, Nobuyuki, Kanagawa (JP)
(74) Representative: Klunker . Schmitt-Nilson . Hirsch

(57) **Abstract**

A medical image diagnosis apparatus (2) which acquires inspection image data by shooting an inspection area and is communicatively connected to an image server (3) that stores the acquired inspection image data and a client terminal (4) in which creation of an inspection report is performed, through a network, in which the medical image diagnosis apparatus includes a storage unit (10) which temporarily stores specific information for specifying an inspection and the inspection image data acquired through the inspection in association with each other, and transmits the inspection image data to the client terminal when the inspection image data is stored in the storage unit in response to a transmission request for the inspection image data of the inspection specified by the specific information being received from the client terminal.

## Description

### BACKGROUND

### 1. Technical Field

The present invention relates to an inspection report creation support system, a medical image diagnosis apparatus, an inspection report creation support method, and a program.

### 2. Related Art

Inspection image data which is acquired by shooting an inspection area using a medical image diagnosis apparatus such as an endoscopic image diagnosis apparatus, an ultrasonic image diagnosis apparatus, or a radiographic image diagnosis apparatus is typically stored in an image server which is communicatively connected to the medical image diagnosis apparatus through a network. Creation of an inspection report is performed in a client terminal which is communicatively connected to the image server through a network, and the inspection image data stored in the image server is transmitted from the image server to the client terminal in response to a request from the client terminal (for example, refer to Patent Literature 1 (JP-A-2005-7145)).

In addition, a system disclosed in Patent Literature 2 (JP-A-2008-6169) is configured such that a medical image diagnosis apparatus and a client terminal in which creation of an inspection report is performed are communicatively connected to each other through a network; and inspection image data which has been acquired using the medical image diagnosis apparatus is temporarily stored in the client terminal, is transmitted from the client terminal to an image server after the creation of the inspection report or the like is completed, and is stored in the image server.

### SUMMARY OF INVENTION

In the system disclosed in Patent Literature 1, for example, in a case where transmission of the inspection image data from the medical image diagnosis apparatus to the image server has not been completed when creation of an inspection report is performed in an early stage after performing an inspection or the like, it is necessary to wait for the completion of transmission. The speed of a network which connects machines in separate facilities is typically lower compared to a network which connects machines in an identical facility. In some cases, the medical image diagnosis apparatus, the client terminal in which creation of an inspection report is performed, and the image server are installed in separate facilities when the size of the system is large or the like. In this case, a considerable time is required before the completion of transmission of the inspection image data from the medical image diagnosis apparatus to the image server. For this reason, there is a concern that the efficiency of creating an inspection report may deteriorate.

In the system disclosed in Patent Literature 2, in a case where there are a large number of medical image diagnosis apparatuses when the size of the system is large or the like, it is necessary that the client terminal is provided with many storage areas sufficient for storing a large amount of inspection image data which is acquired by these medical image diagnosis apparatuses. Accordingly, the system disclosed in Patent Literature 2 is limited to a system with a comparatively small size.

In view of above, an object of the invention is to support efficient creation of an inspection report.
(1) An aspect of the invention provides an inspection report creation support system including: a medical image diagnosis apparatus which acquires inspection image data by shooting an inspection area; an image server which is communicatively connected to the medical image diagnosis apparatus through a network and stores the inspection image data transmitted from the medical image diagnosis apparatus; and a client terminal which is communicatively connected to the medical image diagnosis apparatus and the image server through the network, in which the inspection report creation support system supports creation of an inspection report performed by the client terminal, and the medical image diagnosis apparatus includes a storage unit which temporarily stores specific information for specifying an inspection and the inspection image data acquired through the inspection in association with each other, and transmits the inspection image data to the client terminal when the inspection image data is stored in the storage unit in response to a transmission request for the inspection image data of the inspection specified by the specific information being received from the client terminal.
(2) Another aspect of the invention provides a medical image diagnosis apparatus which acquires inspection image data by shooting an inspection area and is communicatively connected to an image server that stores the acquired inspection image data and a client terminal in which creation of an inspection report is performed, through a network, in which the medical image diagnosis apparatus includes a storage unit which temporarily stores specific information for specifying an inspection and the inspection image data acquired through the inspection in association with each other, and transmits the inspection image data to the client terminal when the inspection image data is stored in the storage unit in response to a transmission request for the inspection image data of the inspection specified by the specific information being received from the client terminal.
(3) Another aspect of the invention provides an inspection report creation support method for supporting creation of an inspection report which is performed in a client terminal that is communicatively connected to a medical image diagnosis apparatus and an image server through a network, using inspection image data which is acquired by shooting an inspection area using the medical image diagnosis apparatus and is stored in the image server that is communicatively connected to the medical image diagnosis apparatus through the network, in which the medical image diagnosis apparatus temporarily stores specific information for specifying an inspection and the inspection image data which is acquired through the inspection in association with each other, and the method includes transmitting a transmission request for inspection image data of an inspection which is specified by the specific information, from the client terminal to the medical image diagnosis apparatus, and transmitting the inspection image data from the medical image diagnosis apparatus to the client terminal when the inspection image data of the specified inspection is stored in the medical image diagnosis apparatus which has received the transmission request.
(4) Another aspect of the invention provides a program causing a computer to execute each of the processes of the inspection report creation support method according to (3).

According to any one of the aspects of the invention, it is possible to support creation of an inspection report and to increase efficiency of creating the inspection report.

### BRIEF DESCRIPTION OF DRAWINGS

Fig. 1 is a view for illustrating an embodiment of the present invention which shows an example of a configuration of an inspection report creation support system.
Fig. 2 is a view showing an example of exchange of information among a medical image diagnosis apparatus, an image server, and a client terminal in the inspection report creation support system in Fig. 1.
Fig. 3 is a view showing an example of exchange of information between the medical image diagnosis apparatus and the client terminal when inspection image data is transmitted from the medical image diagnosis apparatus to the client terminal in the inspection report creation support system in Fig. 1.
Fig. 4 is a view showing an example of a processing flow of the inspection report creation support system in Fig. 1.
Fig. 5 is a view showing another example of the processing flow of the inspection report creation support system in Fig. 1.
Fig. 6 is a view showing another example of exchange of information between the medical image diagnosis apparatus and the client terminal when inspection image data is transmitted from the medical image diagnosis apparatus to the client terminal in the inspection report creation support system in Fig. 1.

### DESCRIPTION OF EMBODIMENTS

Hereinafter, an exemplary embodiment of the present invention will be described with reference to the drawings.

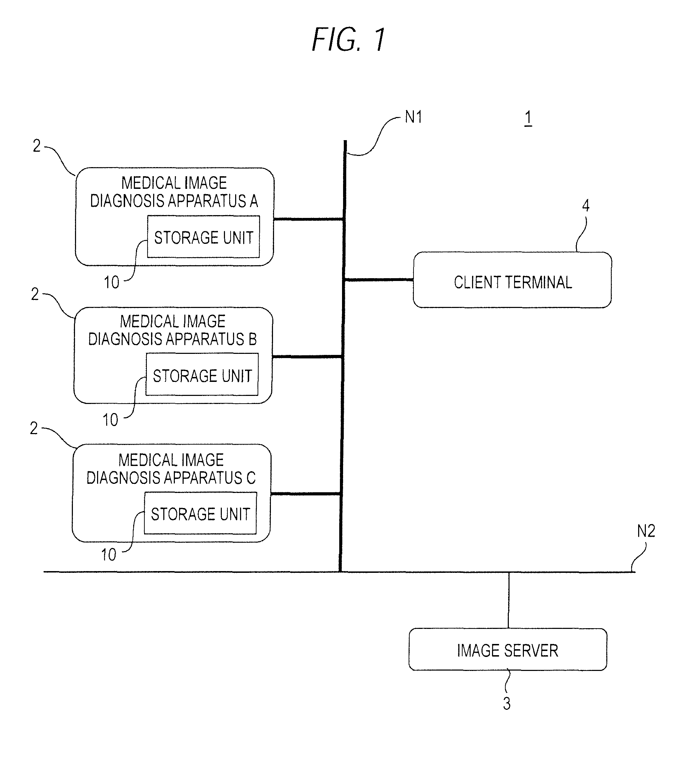

Fig. 1 is a view for illustrating an embodiment of the present invention which shows an example of an inspection apparatus that schematically shows an example of a configuration of an inspection report creation support system.

An inspection report creation support system 1 shown in Fig. 1 includes a medical image diagnosis apparatus 2, an image server 3, and a client terminal 4. The medical image diagnosis apparatus 2, the image server 3, and the client terminal 4 are communicatively connected to each other through a network.

The medical image diagnosis apparatus 2 acquires inspection image data by shooting an inspection area of a patient. Examples thereof include an endoscopic image diagnosis apparatus, an ultrasonic image diagnosis apparatus, or a radiographic image diagnosis apparatus. The inspection image data is configured to include one or a plurality of still images or moving images. Each of the medical image diagnosis apparatuses 2 has a storage unit 10 which temporarily stores the acquired inspection image data.

The image server 3 stores the inspection image data which has been acquired using the medical image diagnosis apparatus 2 and transmitted from the medical image diagnosis apparatus 2. In addition, in the client terminal 4, creation of an inspection report using the inspection image data is performed.

The medical image diagnosis apparatus 2 and the client terminal 4 are placed within a hospital (identical facility), and are connected through a network N1 such as a local area network (LAN) which has a comparatively higher speed. In contrast, the image server 3 which stores the inspection image data stores a large amount of data. Therefore, the image server is placed in a separate facility from the facility such as a data center in which the medical image diagnosis apparatus 2 and the client terminal 4 are placed. Moreover, the medical image diagnosis apparatus 2 and the client terminal 4, and the image server 3 are connected through a network N2 such as a wide area network (WAN) which has a relatively lower speed.

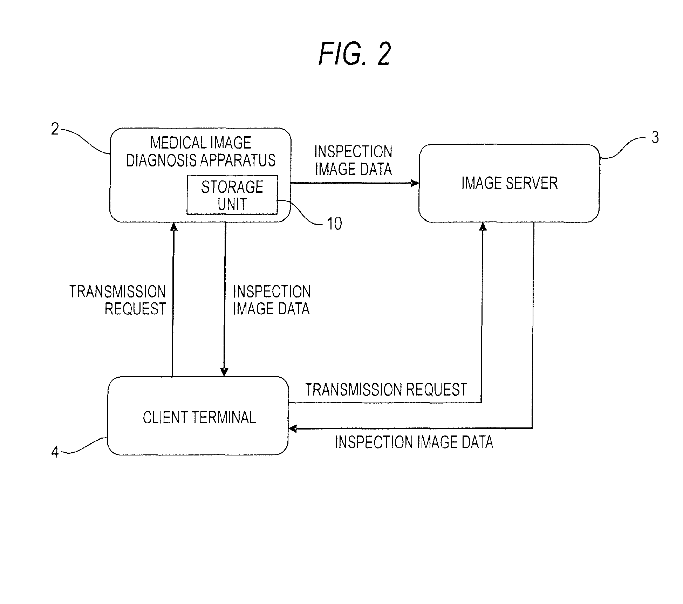

Fig. 2 shows exchange of information among the medical image diagnosis apparatus 2, the image server 3 and the client terminal 4.

The medical image diagnosis apparatus 2, the image server 3, and the client terminal 4 share and use specific information for specifying an inspection. The specific information includes, for example, an identifier for every inspection case, an identifier for every patient, an inspection date and time, and an inspection type (endoscope, ultrasonic waves, radiation, or the like), and in general, is issued by a hospital information system (HIS) including an inspection ordering system or the like for an inspection reservation. In regard to an inspection which has been performed, the medical image diagnosis apparatus 2 temporarily stores inspection image data which has been acquired from the inspection in the storage unit 10 in association with specific information of the inspection.

Then, the medical image diagnosis apparatus 2 transmits the specific information of the performed inspection and the inspection image data which are stored in the storage unit 10 at an appropriate timing, to the image server 3. Examples of the timing of transmitting the specific information and the inspection image data from the medical image diagnosis apparatus 2 to the image server 3 include a nighttime period in which there are generally no orders for inspection. The specific information and the inspection image data stored in the storage unit 10 are deleted from the storage unit 10 at an appropriate timing after the transmission of the specific information and the inspection image data to the image server 3 is completed.

In addition, the medical image diagnosis apparatus 2 is configured so as to be able to receive a transmission request for the inspection image data from the client terminal 4, and transmits the inspection image data to the client terminal 4 when the requested inspection image data is stored in the storage unit 10.

In addition, the image server 3 is also configured so as to be able to receive a transmission request for inspection image data from the client terminal 4, and transmits the inspection image data to the client terminal 4 when the requested inspection image data is stored therein.

The client terminal 4 receives inspection image data of an inspection specified by specific information of the inspection, during which an inspection report is created, from any one of the medical image diagnosis apparatus 2 and the image server 3 using the specific information.

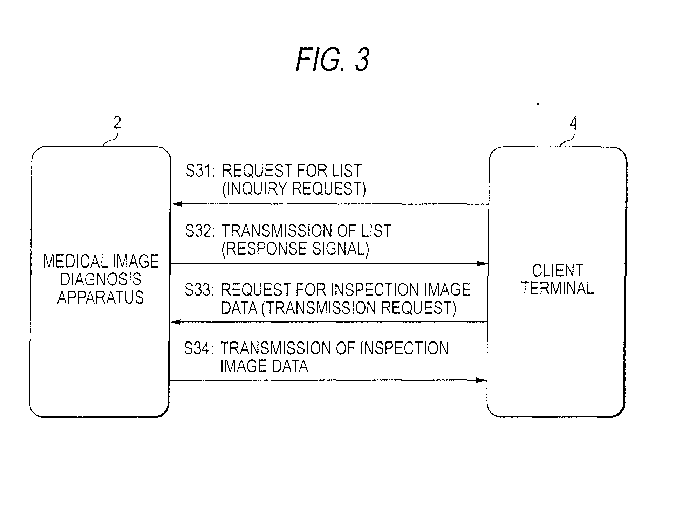

Fig. 3 shows an example of exchange of information between the medical image diagnosis apparatus 2 and the client terminal 4 when inspection image data is transmitted from the medical image diagnosis apparatus 2 to the client terminal 4.

First, the client terminal 4 transmits in step S31 an inquiry request to the medical image diagnosis apparatus 2, to make an inquiry as to whether the inspection image data of the inspection, during which an inspection report is created, is stored in the storage unit 10 of the medical image diagnosis apparatus 2. In this example, the inquiry request is a request for a list of the specific information stored in the storage unit 10. The medical image diagnosis apparatus 2 transmits in step S32 the list of the specific information stored in the storage unit 10, and information of whether an inspection specified by the specific information has been performed for every specific information piece, that is, whether the inspection image data is stored in the storage unit 10 by being associated with the specific information, to the client terminal 4, as response signals with respect to the inquiry request from the client terminal 4.

The client terminal 4 specifies a medical image diagnosis apparatus 2 which is performing or has performed an inspection, during which an inspection report is created, based on the response signal received from each of the medical image diagnosis apparatuses 2. In addition, when the inspection has been performed (when the inspection image data is stored in the storage unit 10 by being associated with the specific information), the client terminal transmits in step S33 specific information of the inspection to the medical image diagnosis apparatus 2 which has performed the inspection as a transmission request for the inspection image data of the inspection. The medical image diagnosis apparatus 2 which has received the transmission request from the client terminal 4 transmits in step S34 the inspection image data which is stored in the storage unit 10 in association with the specific information included in the transmission request to the client terminal 4.

In a case where there are plural medical image diagnosis apparatuses 2, the client terminal 4 may transmit an inquiry request to all of the medical image diagnosis apparatuses 2. However, in a case or the like where it is known which medical image diagnosis apparatus 2 is performing or has performed the inspection, during which an inspection report is created, the client terminal 4 may transmit an inquiry request only to the specific medical image diagnosis apparatus 2 which is performing or has performed the inspection.

In addition, specific information of the inspection, during which an inspection report is created, may be transmitted from the client terminal 4 to the medical image diagnosis apparatus 2 as the above-described inquiry request; retrieval of the specific information received by each of the medical image diagnosis apparatuses 2 may be executed; and information of whether the specific information is stored in the storage unit 10 and whether the inspection image data is stored in the storage unit 10 by being associated with the specific information may be transmitted from each of the medical image diagnosis apparatuses 2 to the client terminal 4 as the above-described response signal.

Exchange of information between the image server 3 and the client terminal 4 when the inspection image data is transmitted from the image server 3 to the client terminal 4 is also performed using the specific information.

First, the client terminal 4 transmits the specific information of the inspection, during which an inspection report is created, to the image server 3, and makes an inquiry as to whether the inspection image data of the inspection is stored in the image server 3. The image server 3 transmits information of whether the received specific information and the inspection image data associated with the specific information are stored as a response signal with respect to the inquiry request from the client terminal 4, to the client terminal 4.

In a case where the inspection image data of the inspection, during which an inspection report is created, is stored in the image server 3, the client terminal 4 transmits the specific information of the inspection to the image server 3 as a transmission request for the inspection image data based on the response signal received from the image server 3. The image server 3 transmits the inspection image data, which is stored in association with the specific information included in the transmission request, to the client terminal 4.

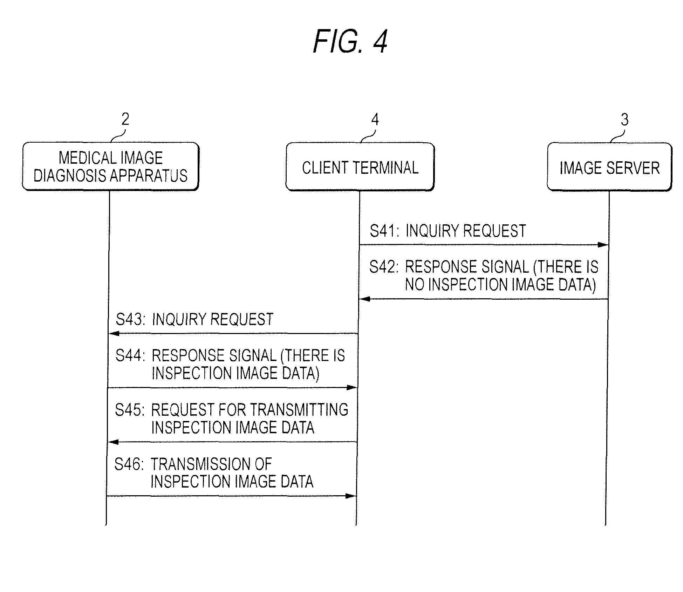

Fig. 4 shows an example of a processing flow of the inspection report creation support system 1.

In the example shown in Fig. 4, it is set such that transmission of inspection image data of an inspection, during which an inspection report is created, from the medical image diagnosis apparatus 2 to the image server 3 is not completed and the inspection image data of the inspection is stored only in the medical image diagnosis apparatus 2.

First, an inquiry request for making an inquiry as to whether the inspection image data of the inspection, during which an inspection report is created, is stored in the image server 3 is transmitted in step S41 from the client terminal 4 to the image server 3. A response signal indicating whether the inspection image data of the inspection is stored in the image server 3 is transmitted in step S42 from the image server 3 to the client terminal 4 in response to the inquiry request from the client terminal 4. The client terminal 4 determines whether the inspection image data of the inspection is stored in the image server 3 based on the received response signal.

In this example, a response signal indicating that the inspection image data of the inspection, during which an inspection report is created, is not stored in the image server 3 is transmitted from the image server 3 to the client terminal 4, and the client terminal 4 determines that the inspection image data of the inspection is not stored in the image server 3.

Next, an inquiry request for making an inquiry as to whether the inspection image data of the inspection, during which an inspection report is created, is stored in the medical image diagnosis apparatus 2 is transmitted in step S43 from the client terminal 4 to the medical image diagnosis apparatus 2. A response signal indicating whether the inspection image data of the inspection is stored in the medical image diagnosis apparatus 2 is transmitted in step S44 from the medical image diagnosis apparatus 2 to the client terminal 4 in response to the inquiry request from the client terminal 4.

In this example, the inspection image data of the inspection, during which an inspection report is created, is stored in the medical image diagnosis apparatus 2. Therefore, a response signal indicating that the inspection image data of the inspection is stored in the medical image diagnosis apparatus 2 is transmitted from the medical image diagnosis apparatus 2 to the client terminal 4.

Then, a transmission request for the inspection image data of the inspection, during which an inspection report is created, is transmitted in step S45 from the client terminal 4 to the medical image diagnosis apparatus 2, and the inspection image data of the inspection is transmitted in step S46 from the medical image diagnosis apparatus 2 to the client terminal 4 in response to the transmission request.

In this manner, in the inspection report creation support system 1, in a case where the inspection image data of the inspection, during which an inspection report is created, is not transmitted from the medical image diagnosis apparatus 2 to the image server 3 when creating the inspection report in the client terminal 4, the inspection image data is directly transmitted to the client terminal 4 from the medical image diagnosis apparatus 2 in which the inspection image data of the inspection is stored. Accordingly, it is possible to perform creation of the inspection report without waiting for the completion of the transmission of the inspection image data from the medical image diagnosis apparatus 2 to the image server 3, and therefore, it is possible to increase the efficiency of creating the inspection report.

The inspection report creation support system 1 is particularly useful when the speed of the network N2 which connects the medical image diagnosis apparatus 2 and the client terminal 4, and the image server 3 is relatively lower than that of the network N1 which connects the medical image diagnosis apparatus 2 and the client terminal 4 and when a considerable time is required for completing the transmission of the inspection image data from the medical image diagnosis apparatus 2 to the image server 3.

When transmitting the inspection image data from the medical image diagnosis apparatus 2 to the client terminal 4, the inspection image data may be compressed for transmission. Accordingly, it is possible to shorten the time required for transmitting the inspection image data from the medical image diagnosis apparatus 2 to the client terminal 4, and therefore, it is possible to further increase the efficiency of creating the inspection report.

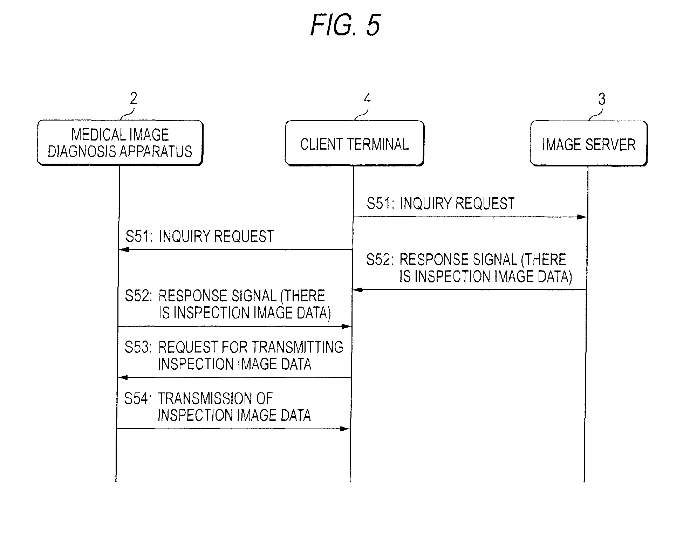

Fig. 5 shows another example of the processing flow of the inspection report creation support system 1.

In the example shown in Fig. 5, it is set such that inspection image data of an inspection, during which an inspection report is created, from the medical image diagnosis apparatus 2 to the image server 3 has been transmitted and remains also in the medical image diagnosis apparatus 2.

First, an inquiry request for making an inquiry as to whether there is inspection image data of an inspection, during which an inspection report is created, is transmitted in step S51 from the client terminal 4 to the medical image diagnosis apparatus 2 and the image server 3. A response signal indicating whether there is inspection image data of the inspection is transmitted in step S52 from the medical image diagnosis apparatus 2 and the image server 3 to the client terminal 4 in response to the inquiry request from the client terminal 4.

In this example, the inspection image data of the inspection, during which an inspection report is created, is stored in both of the medical image diagnosis apparatus 2 and the image server 3. Therefore, response signals indicating that the inspection image data of the inspection is stored therein are respectively transmitted from the medical image diagnosis apparatus 2 and the image server 3 to the client terminal 4.

The client terminal 4 is configured to give priority to the medical image diagnosis apparatus 2 in a case where the client terminal 4 receives the response signals indicating that the inspection image data of the inspection is stored therein from both of the medical image diagnosis apparatus 2 and the image server 3. A transmission request for the inspection image data of the inspection is transmitted in step S53 from the client terminal 4 to the medical image diagnosis apparatus 2, and the inspection image data of the inspection is transmitted in step S54 from the medical image diagnosis apparatus 2 to the client terminal 4 in response to the transmission request from the client terminal 4.

When the speed of the network N1 which connects the medical image diagnosis apparatus 2 and the client terminal 4 is relatively higher than that of the network N2 which connects the medical image diagnosis apparatus 2 and the client terminal 4, and the image server 3, it is possible to shorten the time required for transmitting the inspection image data to the client terminal 4, and therefore, it is possible to increase the efficiency of creating the inspection report.

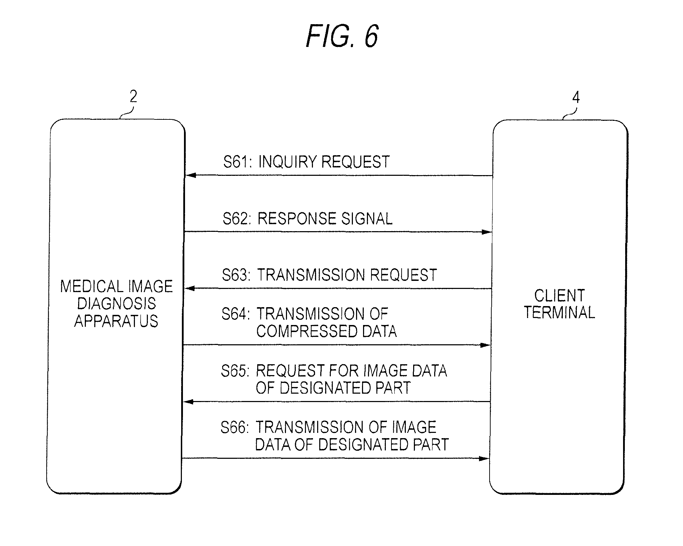

Fig. 6 shows another example of exchange of information between the medical image diagnosis apparatus 2 and the client terminal 4 when inspection image data is transmitted from the medical image diagnosis apparatus 2 to the client terminal 4.

In the example shown in Fig. 6, the inspection image data of the inspection, during which an inspection report is created, is set to a moving image. In the case where the inspection image data is a moving image, an image which is used for creating an inspection report is typically a part of the moving image. In the present example, the time required for transmission is shortened and a network load is reduced by setting the inspection image data (original data), which is transmitted from the medical image diagnosis apparatus 2 to the client terminal 4, to only a part of data which is used for creating an inspection report.

First, an inquiry request for making an inquiry as to whether there is inspection image data of an inspection, during which an inspection report is created, is transmitted in step S61 from the client terminal 4 to the medical image diagnosis apparatus 2. A response signal indicating whether there is inspection image data of the inspection is transmitted in step S62 from the medical image diagnosis apparatus 2 to the client terminal 4 in response to the inquiry request from the client terminal 4. In a case where the response signal indicates that there is inspection image data of the inspection, a transmission request for the inspection image data of the inspection is transmitted in step S63 from the client terminal 4 to the medical image diagnosis apparatus 2.

Next, compressed data of the inspection image data is transmitted in step S64 from the medical image diagnosis apparatus 2 to the client terminal 4 in response to the transmission request for the inspection image data of the inspection, during which an inspection report is created. In the client terminal 4, a part of the inspection image data is designated by a user based on the compressed data.

Then, the transmission request for the designated part of the inspection image data which has been designated by a user is transmitted in step S65 from the client terminal 4 to the medical image diagnosis apparatus 2, and data of the designated part is transmitted in step S66 from the medical image diagnosis apparatus 2 to the client terminal 4 in response to the transmission request.

According to this example, it is possible to shorten the time required for transmission and a network load is further reduced by setting the inspection image data (original data), which is transmitted from the medical image diagnosis apparatus 2 to the client terminal 4, to only a part of data which is used for creating an inspection report.

Although it has been described in this example that the inspection image data of the inspection, during which an inspection report is created, is set to a moving image, the inspection image data is useful also when the inspection image data is configured to include, for example, a plurality of still images or the like. Moreover, a group (compressed data) of thumbnail images of a plurality of still images included in the inspection image data may be transmitted from the medical image diagnosis apparatus 2 to the client terminal 4 and only data (original data) of a part of still images which has been designated by a user may be transmitted from the medical image diagnosis apparatus 2 to the client terminal 4 based on the group of the thumbnail images.

It is also possible to provide an inspection report creation support method which is performed by the above-described inspection report creation support system 1 as a program. In such a program, the program is recorded in a computer-readable non-temporary (non-transitory) recording medium. Such a "computer-readable recording medium" includes, for example, an optical medium such as a Compact Disc-ROM (CD-ROM); or a magnetic recording medium such as a memory card. In addition, such a program can be provided by being downloaded through a network. The above-described program is utilized by, for example, being installed in the client terminal 4 and provides a function of acquiring inspection image data when an inspection report is created.

Hereinafter, the following matter is disclosed in the present specification as described above.
(1) It is an inspection report creation support system including: a medical image diagnosis apparatus which acquires inspection image data by shooting an inspection area; an image server which is communicatively connected to the medical image diagnosis apparatus through a network and stores the inspection image data transmitted from the medical image diagnosis apparatus; and a client terminal which is communicatively connected to the medical image diagnosis apparatus and the image server through the network, in which the inspection report creation support system supports creation of an inspection report performed by the client terminal, and the medical image diagnosis apparatus includes a storage unit which temporarily stores specific information for specifying an inspection and the inspection image data acquired through the inspection in association with each other, and transmits the inspection image data to the client terminal when the inspection image data is stored in the storage unit in response to a transmission request for the inspection image data of the inspection specified by the specific information being received from the client terminal.
(2) The inspection report creation support system according to (1), may have a configuration in which the client terminal determines whether the inspection image data of the specified inspection is stored in the image server and transmits the transmission request to the medical image diagnosis apparatus when the inspection image data of the specified inspection is not stored in the image server.
(3) The inspection report creation support system according to (1), may have a configuration in which the client terminal transmits to the medical image diagnosis apparatus an inquiry request for making an inquiry as to whether the inspection image data of the specified inspection is stored in the storage unit of the medical image diagnosis apparatus and transmits the transmission request preferentially to the medical image diagnosis apparatus in response to a response signal indicating that the inspection image data of the specified inspection is stored in the storage unit of the medical image diagnosis apparatus which has received the inquiry request being received from the medical image diagnosis apparatus.
(4) The inspection report creation support system according to any one of (1) to (3), may have a configuration in which the medical image diagnosis apparatus compresses the inspection image data of the specified inspection and transmits the compressed inspection image data to the client terminal.
(5) The inspection report creation support system according to any one of (1) to (3), may have a configuration in which the medical image diagnosis apparatus transmits compressed data of the inspection image data of the specified inspection to the client terminal, and transmits data of a designated part to the client terminal in a case where the medical image diagnosis apparatus receives a transmission request for the designated part of the inspection image data of the specified inspection which is specified by the client terminal based on the compressed data.
(6) It is a medical image diagnosis apparatus which acquires inspection image data by shooting an inspection area and is communicatively connected to an image server that stores the acquired inspection image data and a client terminal in which creation of an inspection report is performed, through a network, in which the medical image diagnosis apparatus includes a storage unit which temporarily stores specific information for specifying an inspection and the inspection image data acquired through the inspection in association with each other, and transmits the inspection image data to the client terminal when the inspection image data is stored in the storage unit in response to a transmission request for the inspection image data of the inspection specified by the specific information being received from the client terminal.
(7) The medical image diagnosis apparatus according to (6), may have a configuration in which the medical image diagnosis apparatus compresses the inspection image data of the specified inspection and transmits the compressed inspection image data to the client terminal.
(8) The medical image diagnosis apparatus according to (6), may have a configuration in which the medical image diagnosis apparatus transmits compressed data of the inspection image data of the specified inspection to the client terminal, and transmits data of a designated part to the client terminal in response to a transmission request for the designated part of the inspection image data of the specified inspection which is specified by the client terminal based on the compressed data being received from the client terminal.
(9) It is an inspection report creation support method for supporting creation of an inspection report which is performed in a client terminal that is communicatively connected to a medical image diagnosis apparatus and an image server through a network, using inspection image data which is acquired by shooting an inspection area using the medical image diagnosis apparatus and is stored in the image server that is communicatively connected to the medical image diagnosis apparatus through the network, in which the medical image diagnosis apparatus temporarily stores specific information for specifying an inspection and the inspection image data which is acquired through the inspection in association with each other, and the method includes transmitting a transmission request for inspection image data of an inspection which is specified by the specific information, from the client terminal to the medical image diagnosis apparatus, and transmitting the inspection image data from the medical image diagnosis apparatus to the client terminal when the inspection image data of the specified inspection is stored in the medical image diagnosis apparatus which has received the transmission request.
(10) It is a program causing a computer to execute each of the processes of the inspection report creation support method according to (9).

## Claims

1. An inspection report creation support system comprising:
a medical image diagnosis apparatus which acquires inspection image data by shooting an inspection area;
an image server which is communicatively connected to the medical image diagnosis apparatus through a network and stores the inspection image data transmitted from the medical image diagnosis apparatus; and
a client terminal which is communicatively connected to the medical image diagnosis apparatus and the image server through the network,
wherein the inspection report creation support system supports creation of an inspection report performed by the client terminal, and
the medical image diagnosis apparatus includes a storage unit which temporarily stores specific information for specifying an inspection and the inspection image data acquired through the inspection in association with each other, and transmits the inspection image data to the client terminal when the inspection image data is stored in the storage unit in response to a transmission request for the inspection image data of the inspection specified by the specific information being received from the client terminal.

2. The inspection report creation support system according to claim 1,
wherein the client terminal determines whether the inspection image data of the specified inspection is stored in the image server and transmits the transmission request to the medical image diagnosis apparatus when the inspection image data of the specified inspection is not stored in the image server.

3. The inspection report creation support system according to claim 1,
wherein the client terminal transmits to the medical image diagnosis apparatus an inquiry request for making an inquiry as to whether the inspection image data of the specified inspection is stored in the storage unit of the medical image diagnosis apparatus and transmits the transmission request preferentially to the medical image diagnosis apparatus in response to a response signal indicating that the inspection image data of the specified inspection is stored in the storage unit of the medical image diagnosis apparatus which has received the inquiry request being received from the medical image diagnosis apparatus.

4. The inspection report creation support system according to any one of claims 1 to 3,
wherein the medical image diagnosis apparatus compresses the inspection image data of the specified inspection and transmits the compressed inspection image data to the client terminal.

5. The inspection report creation support system according to any one of claims 1 to 3,
wherein the medical image diagnosis apparatus transmits compressed data of the inspection image data of the specified inspection to the client terminal, and transmits data of a designated part to the client terminal in a case where the medical image diagnosis apparatus receives a transmission request for the designated part of the inspection image data of the specified inspection which is specified by the client terminal based on the compressed data.

6. A medical image diagnosis apparatus which acquires inspection image data by shooting an inspection area and is communicatively connected to an image server that stores the acquired inspection image data and a client terminal in which creation of an inspection report is performed, through a network,
wherein the medical image diagnosis apparatus includes a storage unit which temporarily stores specific information for specifying an inspection and the inspection image data acquired through the inspection in association with each other, and transmits the inspection image data to the client terminal when the inspection image data is stored in the storage unit in response to a transmission request for the inspection image data of the inspection specified by the specific information being received from the client terminal.

7. The medical image diagnosis apparatus according to claim 6,
wherein the medical image diagnosis apparatus compresses the inspection image data of the specified inspection and transmits the compressed inspection image data to the client terminal.

8. The medical image diagnosis apparatus according to claim 6,
wherein the medical image diagnosis apparatus transmits compressed data of the inspection image data of the specified inspection to the client terminal, and transmits data of a designated part to the client terminal in response to a transmission request for the designated part of the inspection image data of the specified inspection which is specified by the client terminal based on the compressed data being received from the client terminal.

9. An inspection report creation support method for supporting creation of an inspection report which is performed in a client terminal that is communicatively connected to a medical image diagnosis apparatus and an image server through a network, using inspection image data which is acquired by shooting an inspection area using the medical image diagnosis apparatus and is stored in the image server that is communicatively connected to the medical image diagnosis apparatus through the network,
wherein the medical image diagnosis apparatus temporarily stores specific information for specifying an inspection and the inspection image data which is acquired through the inspection in association with each other, and
the method includes
transmitting a transmission request for inspection image data of an inspection which is specified by the specific information, from the client terminal to the medical image diagnosis apparatus, and
transmitting the inspection image data from the medical image diagnosis apparatus to the client terminal when the inspection image data of the specified inspection is stored in the medical image diagnosis apparatus which has received the transmission request.

10. A program causing a computer to execute each of the processes of the inspection report creation support method according to claim 9.
